# EUROPEAN PATENT APPLICATION

(11) **EP 3 461 562 A1**
(43) Date of publication of application: **03.04.2019**
(21) Application number: 18195831.5
(22) Date of filing: 20.09.2018
(51) Int. Cl.: B06B 1/06

(54) **ULTRASONIC TRANSDUCER USING AEROGEL AS FILLER MATERIAL**

(30) Priority: 29.09.2017 US 201762565326 P; 18.09.2018 US 201816133921
(71) Applicant: Olympus Scientific Solutions Americas Inc., Waltham MA 02453 (US)
(72) Inventor: MACKERTICH-SENGERY, Galestan, State College, PA 168033 (US)
(74) Representative: Noack, Andreas

(57) **Abstract**

Disclosed is an ultrasonic transducer and a manufacturing method thereof. The transducer comprises a piezoelectric composite array having an array of rigid posts made of a piezoelectric material, with kerf spaces between the posts filled with a low density aerogel material.

## Description

### CROSS-REFERENCE TO RELATED APPLICATIONS

This application claims the benefit and priority of U.S. Provisional patent application Serial No. 62565326 filed September 29, 2017 entitled ULTRASONIC MATRIX COMPOSITE TRANSDUCER USING AEROGEL AS FILLER MATERIAL, the entire disclosure of which is incorporated herein by reference.

### FIELD OF THE INVENTION

The invention relates to the manufacture of ultrasonic array probe transducers for non-destructive testing / inspection (NDT/NDI), and more specifically to the use of a synthetic colloidal mixture aerogel for manufacture of a piezoelectric composite transducer having an air-like filler between the ultrasonic posts of the composite.

### BACKGROUND OF THE INVENTION

Ultrasonic probe array transducers are commonly used for NDT/NDI. A transducer typically comprises a composite of an array of polarized piezoelectric ceramic elements, the ceramic usually being in the form of posts, with each post being mechanically supported and acoustically isolated from its neighbors by a filler material which fills kerf spaces between the posts. In transducers of existing practice the filler material is usually a rigid epoxy material.

Electrical activation of the posts in a transducer's composite element causes the posts to expand and contract in the direction of polarization. This expansion and contraction creates a shear wave at the interface between the post and the filler material. The shear wave should be absorbed by the filler material, however the rigid epoxy of existing practice allows transmission of mechanical vibrations in a direction perpendicular to the polarization direction. These mechanical vibrations transmitted through the epoxy are called lateral mode resonances, planar coupling or acoustical cross-talk, and they cause noise in the returned acoustic signal and a reduction of resolution during the inspection.

A further problem with filler materials in existing practice is that some filler materials have high Poisson ratios. As a result, internal stresses are generated within the filler material that oppose the stress applied to the piezoelectric in order to generate sound waves. This leads to performance loss of the transducer.

Yet a further problem with filler materials in existing practice is that their performance degrades in high temperature operation. Typical epoxy materials have continuous operating temperatures in the range of 50 to 200°C, with degradation occurring above 400°C. As a result, existing filler materials are not compatible with high temperature operation.

Yet a further problem with filler materials in existing practice is that rigid epoxy is not flexible, and therefore the transducer cannot easily be made flexible in order to form complex transducer shapes.

An ideal transducer would be one manufactured with no filler material. In other words there would be only air in the gaps between ceramic posts. One advantage of air is that the acoustic impedance difference between ceramic and air is so great that lateral vibrations would be subject to very high transmission loss at the ceramic/air interfaces, so that almost no acoustical energy could be transmitted to a neighboring post. Therefore the received image quality would be improved and acoustic noise would be reduced.

Alternatively, the improved performance with air isolation would allow flexibility in the design of the ceramic dicing pattern. The kerf width or the amount of material removed to create a post is based on the shear velocity of the filler material. Use of air filler would lead to more design opportunities to develop higher performing ceramic patterns. It would also be possible to develop ceramic post patterns that can eliminate grating lobes, which are undesirable off-axis secondary ultrasound beams.

A further advantage of the use of air filler is that air cannot have a Poisson ratio, and therefore there would be minimal material stresses resisting the expansion and contraction of the posts to generate sound waves.

Yet a further advantage of the use of air instead of epoxy is that the acoustic impedance of the transducer can be lowered, which allows easier coupling to low impedance materials. For example, water, which is a common coupling medium, has an acoustic impedance over 20 times lower than raw ceramic, and about 9 times lower than transducers in existing practice. The smaller the acoustic impedance difference between the transducer and the coupling medium, the higher will be the transmission of acoustic energy across the interface. The acoustic impedance of water is significantly less than 9 times lower than the acoustic impedance of an air-filled transducer, allowing improved acoustic transmission with the air-filled transducer compared with transducers in existing practice.

Oakley (PCT patent publication WO 90/16087) discloses a piezoelectric device with air-filled kerf. However, Oakley's manufacturing method is to bond the piezoelectric elements directly to electrodes. While this method may be applicable to large piezoelectric posts within an element, it is clearly not possible to bond a micron-sized post to an electrode without support of some kind of matrix.

Bhardwaj (US patent 7,125,468) discloses a gas matrix composite transducer, but the manufacturing method requires the rods to be mechanically stable enough to be placed in rows on sheets of adhesive. Another layer of fabric or adhesive material is placed to separate the next row of rods, until a stack is created. The stack is plated and then the separating layers are removed to create a gas filled matrix. This method requires the rods be at the proper length and their physical location in relation to the neighboring posts to be very accurate. However, it should be noted that at ultrasound operating frequencies in present use, rod separation of as little as 15 microns is required. Achieving accurate placement of rods with such small separation is clearly very difficult and Bhardwaj's method will not be manufacturable.

Therefore there exists a need to provide a manufacturable method of making ultrasonic transducers having filler material which is as close as possible to air only.

### SUMMARY OF THE INVENTION

Accordingly, it is a general objective of the present disclosure to provide an ultrasonic probe transducer with a filler that is nearly true air-filled and a manufacturing method thereof, wherein the transducer and the manufacturing method are suitable for micron-sized structures.

It is further an objective of the present disclosure to provide a nearly true air-filled ultrasonic probe array transducer in which acoustic performance is improved by reducing lateral mode resonances and reducing acoustic crosstalk.

It is further an objective of the present disclosure to provide a nearly true air-filled ultrasonic probe array transducer which has better performance at elevated temperature.

It is further an objective of the present disclosure to provide a nearly true air-filled ultrasonic probe array transducer which has lower acoustic impedance.

It is further an objective of the present disclosure to provide a filler material which may be customized to be more rigid or flexible depending on the application.

These objectives are achieved by utilizing a filler which is a precursor of an aerogel type material that has the mechanical properties required to stabilize the ceramic posts in place. After filling, the aerogel precursor may be dried either using evaporative (sub-critical) drying or using the technology of supercritical drying . After drying, the ceramic posts are supported by filler material with a low density, high strength dendritic microstructure which closely approximates all the desirable properties of air.

### BRIEF DESCRIPTION OF THE DRAWINGS

Fig. 1A is a schematic diagram of a piezoelectric ceramic which has been diced.
Fig. 1B is a schematic diagram of a piezoelectric ceramic in which the kerf spaces have been filled by a filler material.
Fig. 1C is a schematic diagram of a ceramic composite in which the base of the piezoelectric ceramic has been ground away.
Fig. 1D shows the components of an ultrasonic transducer.
Fig. 2 is a phase of diagram illustrating different methods of drying.
Fig. 3 is a micrograph showing the dendritic structure of a graphitic aerogel.
Fig. 4A is a flow diagram of a manufacturing method in existing practice.
Fig. 4B is a flow diagram of a manufacturing method according to the present disclosure.

### DETAILED DESCRIPTION OF PREFERRED EMBODIMENT

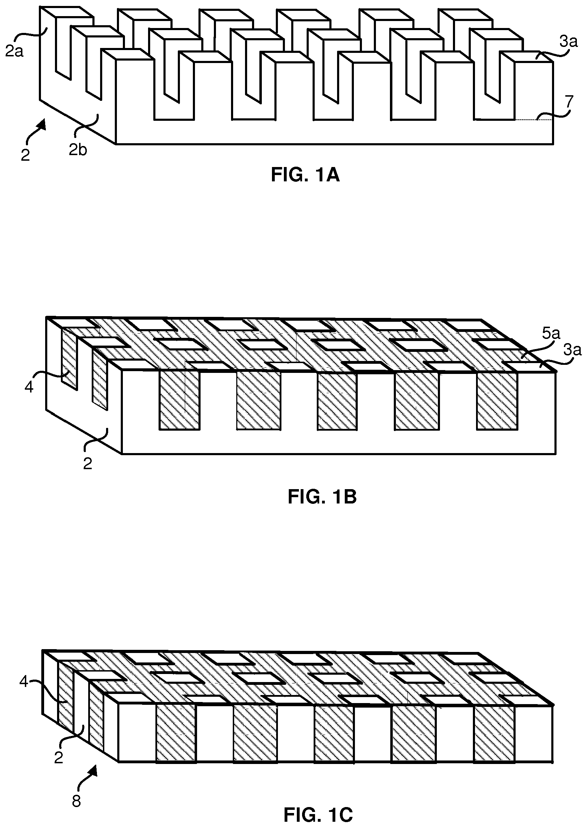

Figs. 1A, 1B and 1C schematically illustrate an existing typical transducer manufacturing process. Fig. 1A shows a solid piezoelectric ceramic block **2**, comprising a diced upper block section **2a** and a lower block section **2b**, which is not diced. Upper block section **2a** is diced into a grid, thereby forming an array of posts having top post surfaces **3a**, the posts being mechanically supported by the material of lower block section **2b** at lower connections **7**. Note that there may be thousands of posts, and in current practice typical dimensions of each individual post may range from 30-400 microns wide by 300 to 1000 microns tall, with kerf spacing between these individual posts ranging from 15 to 250 microns.

In Fig. 1B, the kerf spaces of the grid have been filled by a filler material **4** having a top filler surface **5a**. The purpose of filler material **4** is to mechanically support and acoustically isolate each post. In existing practice, filler material **4** is a rigid epoxy material which is slow cured to reduce contraction of the epoxy, which could damage the posts by deforming or fracturing them. In Fig. 1C, the base of piezoelectric ceramic **2** is ground away, exposing bottom post surfaces **3b** (not shown) and a bottom filler surface **5b** (not shown). The remaining structure after grinding is a piezoelectric composite array **8** which comprises an array of piezoelectric posts supported by the epoxy matrix.

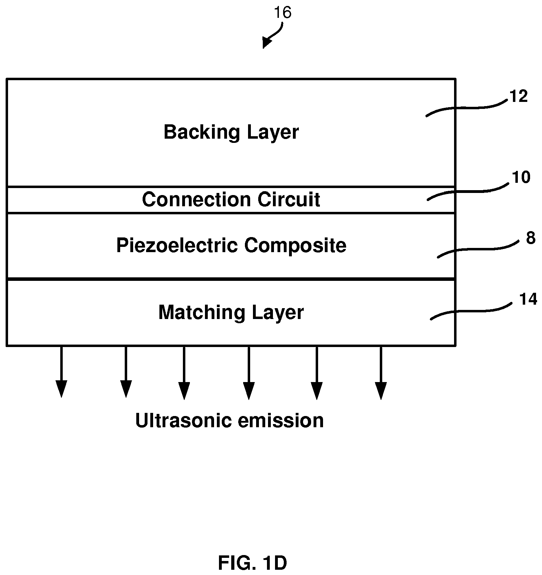

Fig. 1D is a schematic illustration of an ultrasonic transducer **16**. Ultrasonic transducer **16** comprises piezoelectric composite array **8**, a connection circuit **10**, a backing layer **12** and a matching layer **14**. Connection circuit **10**, connected to the non-emitting side of piezoelectric composite array **8**, comprises a circuit board or flexible ribbon having a metallization pattern configured to make electrical contacts with top post surfaces **3a**, and to interface the electrical contacts to a multi-conductor cable (not shown). Backing layer **12** is attached to connection circuit **10**, and comprises a material configured to damp the ultrasonic vibrations thereby preventing emission from that side of piezoelectric composite array **8**. Matching layer **14** is configured to match the acoustic impedance of piezoelectric composite array **8**, thereby maximizing transmission and minimizing reflections of acoustic energy from the emitting side of piezoelectric composite array **8**.

In a piezoelectric composite array according to the present disclosure, the rigid epoxy filler material of existing practice is replaced by an aerogel material. An aerogel is a synthetic porous ultralight material (density less than 10mg/cm³) which is derived from a gel in which the liquid component of the gel has been replaced with a gas. Aerogels created from silica, carbon and metal oxide are known in the art.

Silica aerogels are typically synthesized using a sol-gel process or homogenous solution, in which a colloidal suspension of solid particles ("sol") is hydrolyzed to form a gel having a jellylike consistency. The gel has a liquid component which contains a network of solid bridge material linking the colloidal particles. Finally, during the drying process of the aerogel, the liquid surrounding the network is carefully removed and replaced with air, while keeping the aerogel intact. The resulting aerogel filler material may comprise greater than 96% air. However the remaining micro-dendritic structure within the filler provides stabilization of the micron-sized posts of piezoelectric ceramic.

An aerogel may be dried either using evaporative (sub-critical) drying, freeze drying, or using the technology of supercritical drying. Supercritical drying is used in cases where evaporative or subcritical drying would cause the aerogel structure to collapse on itself due to internal stresses caused by surface tension of the liquid-gas interface. Supercritical drying allows creation of lower density (higher porosity) aerogels, but usually requires high pressure and multiple solvents. Subcritical or evaporative drying is typically used for higher compressive strength aerogels or aerogels that need enhanced hydrophobicity. Subcritical or evaporative drying requires multiple solvents and specialized preparations and may create slightly higher density aerogels than supercritical drying. However, aerogels having porosity as high as 95% may be produced.

Supercritical drying, also known as critical point drying, may be used to achieve aerogels with high porosity and low density. In supercritical drying, the liquid in the aerogel is replaced by a carrier fluid which is forced into a supercritical fluid state by increasing the temperature and pressure. By subsequently dropping the pressure, the carrier fluid is instantly gasified and thereby removed from the aerogel.

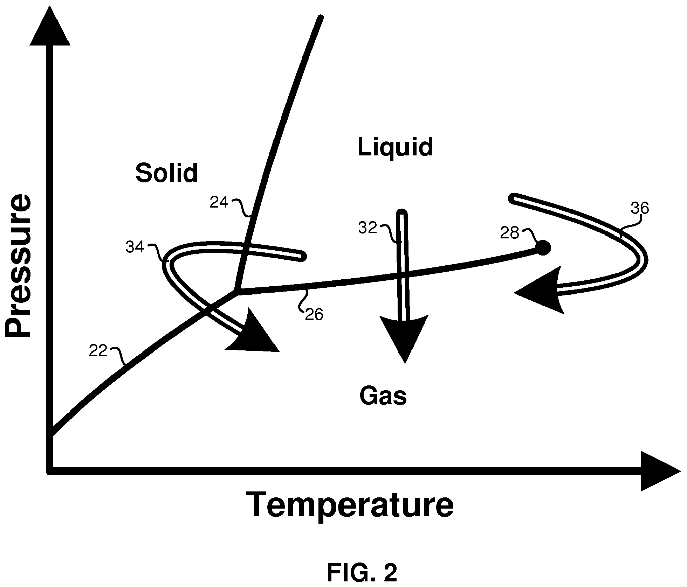

Fig. 2 is a phase diagram illustrating various drying methods. In Fig. 2, a line **22** represents the transition between solid and gas, a line **24** represents the transition between solid and liquid, and a line **26** represents the transition between liquid and gas. Conventional evaporative drying is represented by an arrow **32**, which crosses the liquid/gas boundary. However, the surface tension forces in evaporative drying may cause damage to the micro-dendritic aerogel structures. An arrow **34** represents freeze drying, which may also damage delicate micro-structures at the solid/gas boundary. Supercritical drying is represented by an arrow **36**. In this case, the drying occurs at high temperature and pressure such that the material passes through a supercritical region delineated by a critical point **28**, which is the condition of temperature and pressure at which the liquid and gas densities are equal. Thus the critical point drying process avoids phase transitions which may result in damaging forces. Since the critical point of water occurs at inconveniently high temperature and pressure (347°C and 3,212psi), the water content may be replaced by carbon dioxide (critical point at 31°C and 1,072psi) as a carrier fluid for the purpose of supercritical drying.

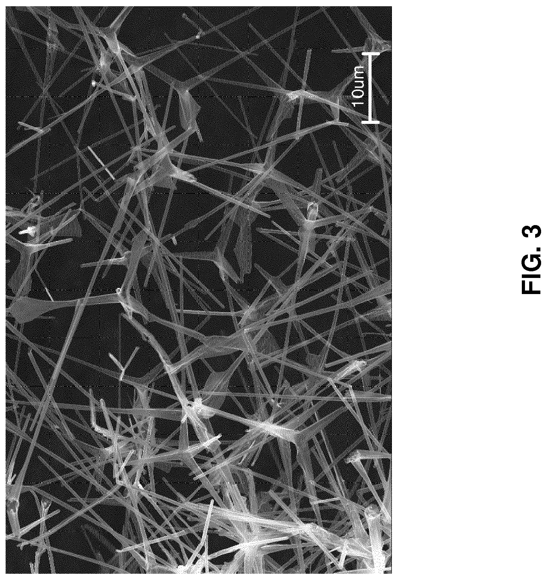

Fig. 3 is a micrograph of the dendritic structure of an exemplary aerogel filler material after the drying process has been completed. The example shown in Fig. 3 is of a carbon-based aerogel made from carbon microtubes and having a density of less than 0.00018 g/cm³.

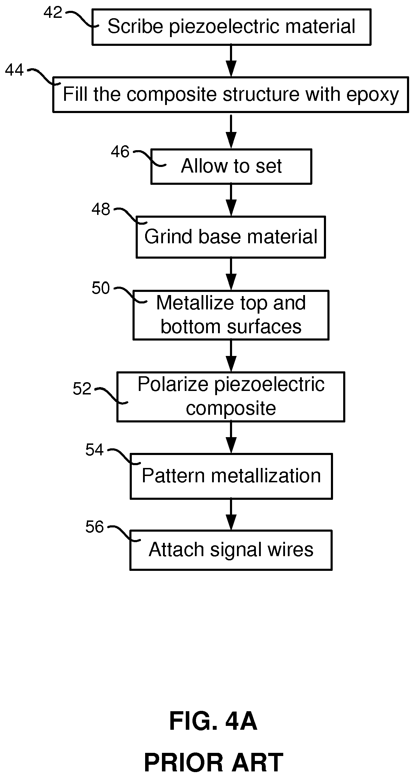

Fig. 4A is a flowchart of a method in existing practice of manufacturing a piezoelectric composite transducer. In step **42**, piezoelectric ceramic block **2** is scribed to form diced upper block **2a** as illustrated in Fig. 1A, thereby forming a multiplicity of ceramic posts. In step **44**, the kerf spaces between the posts are filled with epoxy filler material **4** as illustrated in Fig. 1B, and in step **46** the epoxy is allowed to set. In step **48** the base material of the ceramic is ground away as illustrated in Fig. 1C. In step **50**, the top and bottom surfaces of the composite are metallized to allow polarization of the piezoelectric material in step **52**. Polarization step **52**, depending on the type of piezoelectric material, may comprise application of suitable voltage between the top and bottom surfaces of the composite. In step **54**, the metallization is patterned by etching or other means, so that each individual ceramic post is metallized, and in step **56** signal wires and ground wires are attached to each ceramic post.

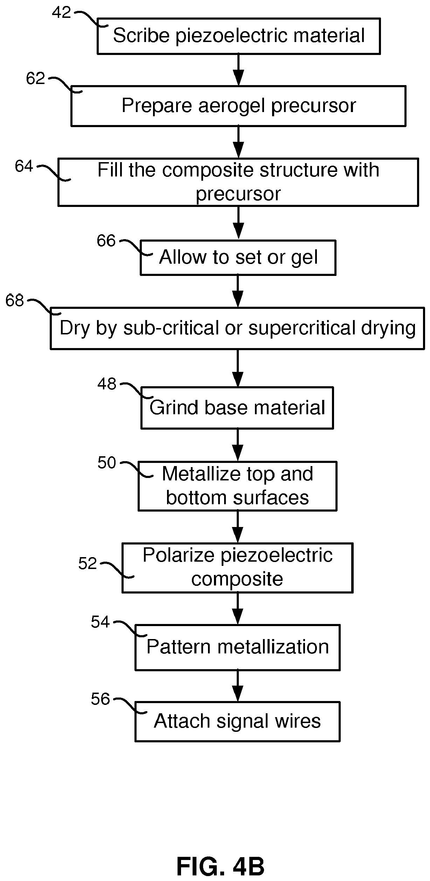

Fig. 4B is a flowchart of a method according to the present disclosure of manufacturing a piezoelectric composite transducer having aerogel filler material. Note that the manufacturing steps of Fig. 4B may be performed using any piezoelectric material, including, but not limited to, lead titanate, barium titanate and lithium niobate. Step **42**, scribing the piezoelectric material, is the same as in existing practice. In step **62**, filler material **4** comprising an aerogel precursor, such as a colloidal mixture or sol-gel of an aerogel material having the desired final properties, is prepared. In step **64**, the kerf spaces between the posts in the composite structure are filled with the aerogel precursor. The precursor is designed to have low enough viscosity to allow the precursor to flow into the kerf spaces. After allowing or causing the precursor to set or gel in step **66**, the mixture is dried in step **68**, either by sub-critical or by supercritical drying or by freeze drying, depending on the aerogel formulation. After drying, the resultant aerogel dendritic structure is sufficiently strong to support micron-sized posts and therefore the remaining manufacturing steps may be the same as in existing practice, namely grinding step **48**, metallizing step **50**, polarizing step **52**, patterning step **54** and attaching signal wires step **56**.

Aerogels which may be suitable for the above manufacturing process include, but are not limited to the following:
a. Silica aerogels, such as those available from Aerogel Technologies Inc.
b. Carbon aerogels, such as Aerographite or Aerographene. See, for example, Mecklenburg et al, "Aerographite: Ultra Lightweight, Flexible Nanowall, Carbon Microtube Material with Outstanding Mechanical Performance", Advanced Materials. 24, 3486-3490, (2012)
c. Metal oxide aerogels, including aluminum oxide, nickel-aluminum oxide, iron oxide, chromium oxide and zirconium oxide.
d. Metal Aerogels.
e. Nanotube Aerogels. See, for example, B. Zheng, Y. Li, J. Liu, "CVD synthesis and purification of single-walled carbon nanotubes on aerogel-supported catalyst", Applied Physics A, 74, 345-348 (2002).
f. Metal Chalcogenide Aerogels. See, for example, Jaya L. Mohanan et al, "Porous Metal Chalcogenide Aerogels", Science 307, 397-400 (2005).
g. Biofoam. See, for example, US Patents 5,382,285 and 5,360,828

**TABLE 1**

| **Material** | **EPO-TEK 301 Epoxy** | **Silica Aerogel** |
|---|---|---|
| Density | ∼ 1.2 g/cm³ | .001 to 0.35 g/cm³ |
| Sound Speed | ∼ 2.6 mm/µsec | <= 0.07 to 1.3 mm/µsec |
| Dielectric Constant | ∼ 4.0 | 1.008 to 2.3 |
| Continuous Operating Temperature | ∼55 to 200 C | Up to 1000 C |
| Degradation Temp | 430°C | >1200°C |

Table 1 compares the properties of a commonly used filler epoxy in current practice (Epo-Tek 301) with the range of properties exhibited by silica aerogels. Of particular note is the low density of the aerogel, the low sound velocity and the high continuous operating temperature and degradation temperature. The aerogel properties shown in Table 1 are particularly desirable for use as a filler material.

Although the present invention has been described in relation to particular embodiments thereof, it can be appreciated that various designs can be conceived based on the teachings of the present disclosure, and all are within the scope of the present disclosure.

## Claims

1. An ultrasonic transducer comprising:
a layer of ultrasonic composite having an emitting face and a non-emitting face, the layer of ultrasonic composite further comprising:
an array of posts made of a piezoelectric material, each of the posts having two endings, one ending being a post connection surface and the other ending partially forming the emitting face, and, each of the posts being surrounded at least partially by neighboring posts separated by kerf spaces; and,
a filler material filling the kerf spaces between the posts, wherein the filler material is an aerogel material;
a layer of connection circuit having a first face with an array of conductive contacts, each conductive contact making electrical contact with a corresponding post connection surface; and
a backing layer attached to a second face of the layer of connection circuit, the backing layer substantially preventing acoustic emission from the non-emitting face.

2. The transducer of claim 1 wherein each of the posts has a post height between 300 and 1000 microns and a post width between 30 and 400 microns, and the kerf spaces have a kerf spacing between 15 and 250 microns.

3. The transducer of claim 1 or 2 wherein the aerogel material is or comprises one or more of a silica aerogel, a carbon aerogel, a metal oxide aerogel, a metal aerogel, a nanotube aerogel, a metal chalcogenide aerogel, and/or a biofoam material.

4. The transducer of any of claims 1 to 3 wherein the aerogel material has a density between 0.001 and 0.35 g/cm³.

5. The transducer of any of claims 1 to 4 wherein the aerogel material has a degradation temperature greater than 1000°C.

6. A method of manufacturing an ultrasonic transducer comprising the steps of:
providing a layer of ultrasonic composite having an emitting face and a non-emitting face, which further comprises the steps of,
forming an upper block section of a piezoelectric ceramic block to form an array of posts, leaving an unworked lower block section, wherein neighboring posts are separated by kerf spaces, the posts having top post surfaces and lower connections to the lower block section;
filling the kerf spaces with a filler material comprising an aerogel precursor material, the filler material having a top filler surface after the filling;drying the filler material to form an aerogel filler material;
removing the lower block section, thereby exposing bottom post surfaces and a bottom filler surface;
providing a layer of connection circuit having a first face with an array of conductive contacts, each conductive contact making electrical contact with a corresponding post connection surface; and,
providing a backing layer attached to a second face of the layer of connection circuit, the backing layer substantially preventing acoustic emission from the non-emitting face.

7. The method of claim 6 comprising the additional steps of:
applying a top metal to cover the top post surfaces and the top filler surface with a top metallization;
applying a bottom metal to cover the bottom post surfaces and the bottom filler surface with a bottom metallization;
polarizing the upper block section by applying a voltage between the top metallization and the bottom metallization;
patterning the top metallization to remove the top metal from the top filler surface; and,
patterning the bottom metallization to remove the bottom metal from the bottom filler surface.

8. The method of claim 6 or 7 comprising the additional step of attaching a matching layer to the bottom post surfaces and the bottom filler surface, the matching layer configured to maximize an acoustic transmission from the bottom post surfaces into a test object.

9. The method of any of claims 6 to 8 further comprising a step of allowing the aerogel precursor material to set after the step of filling and before the step of drying.

10. The method of any of claims 6 to 9 further comprising a step of applying chemicals to cause the aerogel precursor material to set after the step of filling and before the step of drying.

11. The method of any of claims 6 to 10 wherein the step of drying the filler material comprises evaporative drying, supercritical drying, and/or freeze drying.

12. The method of any of claims 6 to 11 wherein the filler material is a liquid or a gel.

13. The method of any of claims 6 to 11 wherein the aerogel filler material comprises a dendritic structure.

14. The method of claim 13 wherein the posts are supported by the dendritic structure after completion of the step of removing the lower block section.

15. An ultrasonic composite array comprising:
an array of posts made of a piezoelectric material, each of the posts being surrounded at least partially by neighboring posts separated by kerf spaces; and,
a filler material filling the kerf spaces between the posts, wherein the filler material is an aerogel material.
